# FASCICULE DE BREVET EUROPEEN

(11) **EP 2 604 338 B1**
(45) Date de publication et mention de la délivrance du brevet: **17.01.2018**
(21) Numéro de dépôt: 12290391.7
(22) Date de dépôt: 14.11.2012
(51) Int. Cl.: B01J 23/75, B01J 37/16, B01J 37/18, C10G 2/00, B01J 23/89

(54) **Procédé de fabrication d'hydrocarbures avec chargement en continu du catalyseur**
Herstellungsverfahren von Kohlenwasserstoffen mit kontinuierlicher Aufladung des Katalysators
Method for producing hydrocarbons with continuous loading of the catalyst

(30) Priorité: 14.12.2011 FR 1103861
(43) Date de publication de la demande: 19.06.2013
(73) Titulaire: IFP Energies nouvelles, 92500 Rueil-Malmaison (FR)
(72) Inventeur: Marion, Marie-Claire, 69390 Vernaison (FR); Diehl, Fabrice, 69003 Lyon (FR); Hugues, François, 69390 Charly - Vernaison (FR)

(56) Documents cités:
- WO-A1-2010/049715
- US-A1- 2003 144 130
- US-A1- 2003 144 367
- US-A1- 2007 135 527

## Description

L'invention se rapporte à un procédé de fabrication d'hydrocarbure. En particulier le procédé de fabrication est un procédé Fischer-Tropsch de synthèse d'hydrocarbure à partir de gaz de synthèse.

### État de la technique

La synthèse d'hydrocarbures à partir d'un mélange constitué de CO et H₂, plus communément appelé gaz de synthèse, est connue depuis longtemps. On peut citer en particulier les travaux de F. Fischer et H. Tropsch qui, dès 1923, ont donné leur nom à cette transformation chimique, bien connue sous le nom de synthèse Fischer-Tropsch. La synthèse Fischer-Tropsch (FT) est une réaction qui permet de synthétiser des hydrocarbures liquides paraffiniques, oléfiniques et/ou des dérivés oxygénés à partir de gaz de synthèse, lui-même obtenu à partir de gaz naturel, charbon ou biomasse. Cette réaction, exploitée industriellement en Europe pendant la seconde guerre mondiale et également en Afrique du Sud depuis les années 50, a retrouvé un spectaculaire regain d'intérêt depuis les années 80-90 suite aux évolutions des coûts du pétrole et du gaz mais aussi pour des aspects environnementaux. On peut citer par exemple qu'actuellement de nombreux projets GTL (de l'anglais "gas to liquid") voient le jour, par exemple au Qatar.

La synthèse Fischer-Tropsch (FT) est également une voie de valorisation du gaz naturel et permet, entre autre, de produire des carburants diesel de très bonne qualité et sans soufre à partir de gaz naturel.

De nombreux métaux peuvent être utilisés pour catalyser cette réaction, dont le cobalt (Co) et le fer (Fe). Le catalyseur est généralement préparé par imprégnation d'un support (par exemple un support à base d'alumine, de silice ou de silice alumine...) à partir d'un précurseur du métal, tel qu'un nitrate ou un acétate dudit métal. On obtient après une ou plusieurs étapes de séchage et de calcination le catalyseur dit "sous forme oxyde" appelé aussi "précurseur oxyde du catalyseur" qui comprend l'oxyde métallique supporté sur le support.

Etant donné que la synthèse Fischer-Tropsch est catalysée par le métal et non l'oxyde. Il convient donc ensuite de réduire le précurseur de catalyseur afin de transformer l'oxyde métallique (par exemple Co₃O₄) en phase métallique (Co(0)).

Généralement, la réduction du précurseur de catalyseur est réalisée dans une unité dédiée, sous atmosphère gazeuse en présence par exemple d'hydrogène. Après cette phase de réduction, le catalyseur est de préférence protégé de l'air afin d'éviter sa ré-oxydation et conserver ainsi la phase active métallique en l'état. Un des procédés utilisés industriellement pour protéger le catalyseur réduit durant son stockage et son transport jusqu'au chargement dans l'unité FT est un processus d'enrobage du catalyseur réduit sous paraffine. Dans des conditions de température suffisantes pour que les paraffines soient en phase liquide, (comprise généralement entre 50 et 200 °C), le catalyseur réduit est mélangé à ce liquide. Après refroidissement à température ambiante, le catalyseur se retrouve enrobé et protégé notamment de l'air par les paraffines solidifiées. Ces étapes (réduction et enrobage) sont coûteuses (coût des paraffines d'enrobage, coût de chaque opération unitaire). En outre, l'étape d'enrobage augmente le volume de catalyseur et engendre ainsi un surcoût au niveau du transport.

On connaît le document EP 593 522 qui décrit un procédé d'activation ou de réactivation d'un catalyseur à base de cobalt dans lequel on réduit le catalyseur oxydé (ou précurseur du catalyseur) en présence d'un gaz réducteur comprenant du monoxyde de carbone (CO) et éventuellement de l'hydrogène (H₂) qui doit être présent à une quantité inférieure à 30% en volume d'hydrogène.

Ce même document divulgue un procédé de démarrage d'une unité de synthèse FT mettant en oeuvre une réduction in-situ du catalyseur. Ce procédé comprend les étapes successives suivantes:
i) réduction, dans le réacteur de synthèse Fischer-Tropsch même, du précurseur de catalyseur contenant du cobalt à température élevée avec un gaz contenant du monoxyde de carbone, ce gaz contenant moins de 30% en volume l'hydrogène basée sur le volume de monoxyde de carbone;
ii) passage d'un gaz de synthèse sur le catalyseur réduit, à une température qui est au moins 10°C supérieure à la température maximale atteinte au cours de l'étape ultérieure réalisée sous des conditions Fischer-Tropsch; et
iii) passage du gaz de synthèse sur le catalyseur dans les conditions du procédé Fischer-Tropsch dans le réacteur de synthèse Fischer-Tropsch.

Par ailleurs il a été observé qu'au cours de la synthèse FT une partie du catalyseur et/ou le métal actif peuvent être entrainés dans la phase hydrocarbure formée, suite à des phénomènes d'attrition ou de dissolution ou que le catalyseur lui-même se désactive.

Il est donc nécessaire de faire régulièrement un appoint en catalyseur "frais" pour maintenir la productivité de l'unité. Or pour que le procédé FT soit rentable industriellement, la mise à l'arrêt de l'installation, par exemple pour faire l'appoint en catalyseur, doit être la moins fréquente possible. En effet toute interruption de l'unité est délicate et coûteuse en temps donc en argent.

Le procédé divulgué dans EP 593 522, qui met en oeuvre une réduction in-situ du précurseur du catalyseur directement dans le réacteur de synthèse FT, n'est donc pas une solution au problème de fournir un procédé dont les arrêts, en particulier pour approvisionnement en catalyseur, sont moins fréquents et qui soit rapidement "opérationnel" après une mise à l'arrêt.

### Résumé de l'invention

Un but de l'invention est de proposer un procédé de synthèse d'hydrocarbure qui soit plus avantageux du point de vue de la gestion du flux de matières (e.g. transport, stockage) et moins coûteux à mettre en oeuvre, notamment en évitant une réduction ex-situ du catalyseur et donc l'étape de protection du catalyseur une fois réduit.

Un autre but de l'invention est de proposer un procédé pour lequel l'approvisionnement en catalyseur réduit ne nécessite pas un arrêt total du réacteur FT ou une interruption de la production.

A cette fin, il est proposé un procédé de fabrication en continu d'hydrocarbures à partir de gaz de synthèse en présence d'un catalyseur comme défini dans la revendication 1. Dans le cadre de l'invention, le "précurseur de catalyseur" peut comprendre, outre l'oxyde de cobalt, un ou plusieurs autres métaux ou oxyde mixte agissant comme des éléments promoteurs, mais également ce terme désigne une phase comprenant l'oxyde mentionné ci-dessus et supporté sur un support de catalyse.

Ainsi selon l'invention, l'étape de réduction du précurseur de catalyseur est intégrée in-situ au procédé de synthèse FT proprement dit, ce qui permet de réduire et d'activer le précurseur du catalyseur sur le site même de l'unité de synthèse FT et donc de supprimer les coûts engendrés par la protection, le stockage et le transport du catalyseur réduit. En outre, la réduction est réalisée dans un réacteur dédié à cet effet ; ceci a pour avantage qu'il n'est plus nécessaire d'arrêter le réacteur de synthèse FT afin de disposer d'une source de catalyseur activé "frais", contrairement au procédé divulgué dans le document EP 593 WO 2010/049715 divulgue un procédé Fischer-Tropsch de fabrication d'hydrocarbures. Un autre but de l'invention est de proposer un procédé dont le démarrage est facilité et dont la durée de la phase transitoire de démarrage est la plus limitée possible.

A cette fin, le procédé inclut en outre les étapes suivantes:
d) chargement d'une quantité utile Q du précurseur du catalyseur à l'état oxydé dans le réacteur de réduction;
e) réduction de la quantité totale Q du précurseur du catalyseur à l'état oxydé chargé à l'étape d) en présence d'un gaz réducteur comprenant du monoxyde de carbone et/ou de l'hydrogène;
f) chargement de la quantité totale Q de catalyseur à réduit à l'étape e) dans le réacteur de synthèse Fischer-Tropsch.

Ainsi selon le procédé, on peut également activer par avance une quantité utile de catalyseur en vue d'un imminent arrêt (par exemple pour maintenance) tout en continuant à faire fonctionner l'unité de synthèse jusqu'au dernier moment avec l'arrêt; là encore on dispose d'une réserve de catalyseur réduit "frais" pour le redémarrage subséquent.

Un autre but de l'invention est de proposer un procédé dont le démarrage est facilité notamment en réduisant autant que possible le temps nécessaire pour que l'activité catalytique du catalyseur atteigne une valeur dite stationnaire correspondant à celle rencontrée dans les conditions stationnaires de synthèse FT. A cette fin, le procédé inclut en outre une étape de conditionnement du catalyseur réduit issu de l'étape b) ou e) en présence d'un gaz comprenant du CO seul ou un mélange CO/H2, avant son chargement dans le réacteur de synthèse Fischer-Tropsch.

Cette étape de conditionnement peut consister à mettre en contact le catalyseur réduit avec un gaz réducteur comprenant un mélange optimisé comportant de l'hydrogène (H₂) et du monoxyde de carbone (CO), dans des conditions de température et de pression adéquates. Par exemple, le conditionnement du catalyseur réduit peut être effectué sous un gaz de synthèse non modifié issu de la même source de gaz de synthèse alimentant le réacteur de synthèse FT.

De manière avantageuse, on peut utiliser le réacteur de réduction comme réacteur de conditionnement du catalyseur déjà réduit afin de former le "vrai catalyseur FT" ("true FT catalyst" en anglais) tel que décrit notamment par H. Schulz (Catal. Today 71, 2002, 351-360).

Le vrai catalyseur est celui qui opère dans le réacteur de synthèse FT. En effet, le catalyseur FT finit de se construire sous gaz de synthèse dans les conditions du procédé. Durant cette phase de fin de construction, l'activité du catalyseur FT se modifie. L'étape de conditionnement permet donc de finir de construire le catalyseur FT par avance en vue de sa mise en oeuvre dans le réacteur de synthèse FT fonctionnant dans des conditions stationnaires.

Il est également possible de réaliser cette étape de conditionnement dans une enceinte dédiée, différente du réacteur de réduction et du réacteur de synthèse FT.

Cette mise en conditionnement du catalyseur réduit peut être également réalisée en plusieurs phases. Par exemple le catalyseur réduit subit une première phase de conditionnement sous gaz de synthèse ayant un premier rapport molaire H₂/CO et ensuite une seconde phase sous gaz de synthèse de rapport molaire H₂/CO différent du premier rapport. Bien entendu, l'homme du métier peut adapter le nombre de phases et les paramètres de conditionnement (e.g. la composition du gaz réducteur, température, la pression totale) en fonction du type de catalyseur et du processus de réduction mis en oeuvre à l'étape b) ou e).

Avantageusement, le gaz réducteur de conditionnement est un gaz de synthèse (i.e. qui comprend un mélange CO/H₂) qui provient directement de la même source de gaz de synthèse alimentant le réacteur FT et dont le rapport molaire H₂/CO est compris entre 0,01 et 10.

De façon préférée, les paramètres opératoires de l'étape de conditionnement seront déterminés de manière à induire également un changement de structure de la phase active, par exemple une transition de phase cristalline du cobalt en phase hexagonale compacte.

De façon alternative, le conditionnement du catalyseur est mis en oeuvre en présence de gaz réducteur comprenant du CO seul.

Selon un mode de réalisation préféré du procédé, le précurseur de catalyseur est d'abord réduit sous H₂ seul, puis est conditionné sous gaz de synthèse ayant un rapport molaire H₂/CO est compris entre 0,1 et 0,5 et enfin soumis à une seconde phase de conditionnement sous gaz de synthèse de rapport molaire H₂/CO compris entre 1 et 2,5. Cette seconde phase de conditionnement est de préférence conduite avec un gaz de synthèse dont le rapport H₂/CO est proche de celui rencontré dans le réacteur de synthèse FT (c'est à dire dont le rapport H₂/CO est compris entre 1 et 3).

Selon un autre mode de réalisation préféré, le précurseur oxyde de catalyseur est d'abord réduit en présence d'un gaz réducteur comprenant H₂ seul, puis est conditionné en présence d'un gaz comprenant du CO seul. Ce catalyseur est ensuite conditionné avec un gaz comprenant le mélange CO et H₂ tel que le rapport molaire H₂/CO soit compris entre 2 et 10. Enfin ce solide est soumis dans un dernière phase à un gaz de synthèse dont le rapport molaire H₂/CO est proche de celui rencontré dans le réacteur de synthèse FT (c'est à dire dont le rapport H₂/CO est compris entre 1 et 3).

Selon un autre mode de réalisation préférée, le précurseur de catalyseur est d'abord réduit en présence d'un gaz de synthèse comprenant un mélange H₂ et CO, tel que le rapport molaire H₂/CO soit supérieur à 1. Puis le catalyseur réduit est conditionné au cours d'une première phase en présence d'un gaz de synthèse comprenant un mélange H₂ et CO, tel que le rapport molaire H₂/CO soit inférieur à 1. Ce solide est enfin soumis à une seconde phase de conditionnement en présence d'un gaz de synthèse dont le rapport molaire H₂/CO est proche de celui rencontré dans le réacteur de synthèse FT (c'est à dire dont le rapport H₂/CO est compris entre 1 et 3).

Selon l'invention, l'alimentation en catalyseur dans le réacteur de synthèse Fischer-Tropsch se fait soit en mode semi-continu soit en mode continue.

De préférence l'alimentation en catalyseur réduit (ou réduit et conditionné) se fait de manière semi-continu. Dans ce mode de fonctionnement seulement une partie du catalyseur qui a été activé (réduit ou réduit et conditionné) est envoyée au réacteur FT. La partie restante du catalyseur activé est maintenue dans des conditions optimales d'activation/conditionnement et peut alors servir de source de catalyseur frais pour un ou plusieurs appoints ultérieurs

Avantageusement le gaz réducteur utile pour la réduction du précurseur de catalyseur comprend de l'hydrogène seul (ou en mélange avec un gaz inerte) ou du monoxyde de carbone seul (ou en mélange avec un gaz inerte) ou un gaz de synthèse présentant un rapport molaire H₂/CO compris entre 0,01 et 10.

Dans le cas où le gaz réducteur est du monoxyde de carbone seul ou un mélange H₂ et CO, ce gaz peut être issu de la source de gaz de synthèse utilisée pour la synthèse FT et dont on a modifié le rapport molaire H₂/CO avant alimentation dans le réacteur de réduction. Par exemple, le traitement du gaz de synthèse peut se faire au moyen d'un dispositif de séparation membranaire en phase gazeuse qui permet d'enrichir le gaz de synthèse en CO.

Dans le cadre de l'invention, la réduction est effectuée à une température comprise entre 200 et 500°C, à une pression totale comprise entre 1 et 50 bar et sous un flux gazeux compris entre 0.1 et 20 Nl/h/g de catalyseur à traiter.

Conformément à l'invention, le précurseur de catalyseur est de l'oxyde de cobalt incorporant éventuellement un ou plusieurs métaux ou oxyde(s) métallique(s), dit "promoteurs", sélectionnés parmi le Cu, Mn, Ru, Pd, Pt, Re, La.

Selon l'invention, l'oxyde de cobalt est supporté par un support comprenant un ou plusieurs oxydes d'un métal choisi parmi Al, Si, Ti, Zr, Ce, Cu, Zn, Ni. On peut citer comme support la silice, l'alumine, la silice-alumine, l'oxyde de titane ou la zircone seule ou en mélange.

### Brève description des figures

Ces aspects ainsi que d'autres aspects de l'invention seront clarifiés dans la description détaillée de modes de réalisation particuliers de l'invention données à titre d'exemple, référence étant faite à l'unique figure, dans laquelle :

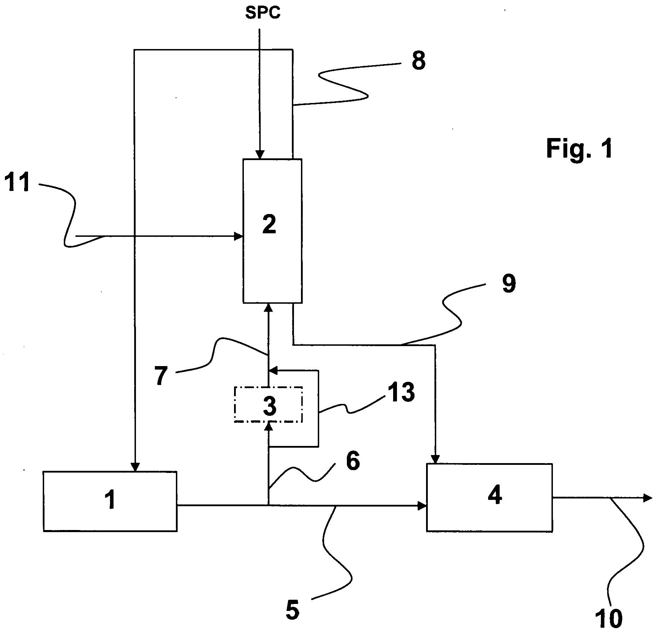

Fig.1 montre un schéma de principe du procédé selon l'invention.

### Description détaillée de modes de réalisation particuliers

La Fig. 1 montre un schéma de principe d'une unité de fabrication d'hydrocarbure par synthèse de type Fischer-Tropch qui comprend:
- un source 1 de gaz de synthèse comprenant comme constituants majoritaires de l'hydrogène (H2) et du monoxyde de carbone (CO);
- un réacteur de réduction 2 alimenté en précurseur du catalyseur FT (catalyseur à l'état oxydé) à partir d'une source en précurseur du catalyseur SPC (non représentée);
- une unité de traitement 3 du gaz de synthèse (cette unité de traitement 3 étant optionnelle);
- un réacteur de synthèse FT 4.

Comme montré sur la Fig. 1, la source 1 de gaz de synthèse alimente le réacteur de synthèse 4 via une conduite d'alimentation 5.

Une conduite 6 permet d'amener une partie du gaz de synthèse depuis la source 1 vers l'unité de traitement 3. Dans le présent exemple, la conduite 6 est connectée à la conduite d'alimentation 5. Cependant il est possible de relier directement la conduite 6 de la source de gaz de synthèse 1.

L'unité de traitement de gaz de synthèse peut être par exemple un dispositif de séparation membranaire gaz/gaz qui permet d'enrichir le gaz de synthèse en monoxyde de carbone CO. Ainsi en sortie de l'unité de traitement 3, le gaz réducteur a un rapport molaire H₂/CO compris entre approximativement 0 et 10, de préférence entre 0,05 et 3, de manière encore préférée entre 0,1 et 1.

La conduite 13 est une conduite de dérivation permettant d'alimenter en gaz de synthèse le réacteur de réduction 2 sans en modifier la composition.

De l'unité de traitement 3 part une conduite 7 destinée à alimenter le réacteur de réduction 2 en gaz réducteur dont la composition a été modifiée.

Dans le cas où la réduction du précurseur de catalyseur est conduite sous hydrogène, le réacteur 2 est alimenté en gaz H₂ (éventuellement dilué dans un gaz inerte), via la conduite 11 qui est elle-même reliée à une source d'hydrogène (non représentée).

Dans le cadre de l'invention, le précurseur de catalyseur comprend de préférence l'oxyde de cobalt et un support à base de silice, alumine, ou silice-alumine, zircone, oxyde de titane ou tout autre support comprenant un ou plusieurs des éléments choisi parmi Al, Si, Ti, Zr, Ce, Co, Cu, Zn, Ni. Le précurseur du catalyseur peut également comprendre un ou plusieurs métaux qui agissent en tant que promoteur. A titre d'exemple, le métal ou les métaux peuvent être sélectionnés parmi Cu, Mn, Ru, Pd, Pt, Re, La.

Lorsque le précurseur de catalyseur comprend de l'oxyde de cobalt et un support, il peut être obtenu par un procédé impliquant une ou plusieurs étape(s) d'imprégnation du précurseur du métal sur le support (par exemple le nitrate de cobalt), suivi d'une étape de séchage et enfin une étape de calcination comme enseigné dans le document EP 527 032. Cette étape de calcination vise notamment à décomposer le précurseur de métal et former l'oxyde métallique.

Le précurseur du catalyseur (cobalt à l'état oxyde), qui est un solide, est mis en contact avec un gaz réducteur par exemple dans un réacteur 2 de type lit fluidisé, lit fixe traversé, lit expansé ou dans une réacteur à contre-courant ou dans un réacteur de type slurry.

Les conditions opératoires pour la réduction sont les suivantes:
- température : comprise entre 200 et 500°C, de préférence entre 200°C et 450°C, de manière encore préférée entre 230 et 400°C ;
- pression totale : comprise entre 0,1 MPa et 5 MPa, de préférence entre 0,1 MPa et 3 MPa ;
- débit du flux gazeux rapporté à la quantité massique de solide catalytique traité : compris entre 0,1 et 20 Nl/h/g, de préférence entre 1 et 10 Nl/h/g;
- durée : comprise entre 1 et 24 h, de préférence entre 1 et 10 h, de préférence entre 2 et 6 h.

L'homme du métier peut optimiser les conditions opératoires de réduction en fonction du type de catalyseur utilisé.

Comme montré sur la Fig. 1, le gaz réducteur effluent est évacué du réacteur de réduction 2 via une conduite 8 et peut éventuellement, et après traitement, être en partie ramené vers la source de gaz de synthèse 1 ou être en partie recyclé vers l'unité 3 ou le réacteur de réduction 2.

Le catalyseur à l'état réduit est quant à lui soutiré du réacteur de réduction 2 et est acheminé vers le réacteur de synthèse FT 4 grâce à la conduite 9.

La réaction de synthèse FT est conduite dans le réacteur 4 dans des conditions opératoires classiques, à savoir:
- température : 200-250°C
- pression totale : 1 - 4 MPa
- rapport molaire H₂/CO : 1 à 3

Les hydrocarbures issus de la réaction FT sont extraits du réacteur de synthèse, via la conduite 10. Une partie des produits formés dans le réacteur 4 est généralement transformée par hydrotraitement (hydrocraquage et hydro-isomérisation) pour produire notamment des carburants.

Dans la suite va être décrit le fonctionnement d'une unité de fabrication mettant en oeuvre le procédé selon l'invention.

### A) phase de démarrage de l'unité

On charge dans le réacteur de réduction 2 une quantité Q utile en précurseur du catalyseur pour une synthèse FT dans le réacteur 4. Parallèlement ou postérieurement on démarre l'unité 1 de production de gaz de synthèse.

On procède ensuite à la réduction de la quantité utile Q de précurseur du catalyseur. A cette fin on introduit dans le réacteur 2 un gaz réducteur qui est, soit le même gaz de synthèse que celui de la source 1 via les conduites (6,13,7), soit un gaz de synthèse ayant subi au préalable un enrichissement permettant d'atteindre quasiment un flux exempt de l'un des 2 constituants (par exemple un flux contenant du CO exempt d'H₂) via les conduites (6,7). Cependant pour des raisons économiques, si une réduction sous hydrogène est envisagée, il est plus intéressant d'utiliser une source externe d'hydrogène qui alimente le réacteur 2 via la conduite 11.

On amène alors le réacteur 2 dans les conditions optimales pour la réduction du précurseur de catalyseur. La réduction peut être optimisée en agissant sur différents leviers tel que le profil de température, la pression, le débit de gaz réducteur.

Une fois la réduction du catalyseur achevée, la quantité totale Q utile est alors introduite dans le réacteur 4 et la réaction de synthèse FT à proprement dite est démarrée tandis que l'on procède à la fermeture de la conduite 9 d'alimentation du catalyseur réduit.

Éventuellement, avant son introduction dans le réacteur FT, le catalyseur réduit subit une phase de conditionnement qui consiste à traiter le catalyseur en présence d'un gaz de synthèse selon une procédure pouvant comprendre plusieurs phases successives mettant en jeu différent paramètres tels que la pureté du gaz de synthèse, le rapport molaire H₂/CO du gaz de synthèse, la température et la pression. Les paramètres opératoires de l'étape de conditionnement sont les suivants:
- Température: 200-500°C, de préférence 200-300°C;
- Pression: 0,1 à 5 MPa, de préférence 1 à 4 MPa;
- Rapport molaire H₂/CO : 0 à 10
- Pureté du gaz de synthèse comprise entre 10 et 100%;
- GHSV: 0.1 à 20 Nl/h/g, de préférence entre 1 et 10 NI/h/g ;
- Durée : comprise entre 1 et 48 h, de préférence entre 2 et 24 h, de préférence entre 2 et 15 h.

### B) phase de fabrication d'hydrocarbure

Après démarrage de la synthèse, le réacteur de réduction 2 est de nouveau alimenté en précurseur du catalyseur et en gaz réducteur afin de préparer du catalyseur activé "frais" qui servira par la suite à approvisionner le réacteur FT 4 pendant le temps de fonctionnement en continu dudit réacteur 4.

De manière préférée, le catalyseur une fois réduit subit une phase de conditionnement dans lequel il est traité en présence d'un gaz de synthèse selon une procédure mettant en oeuvre une ou plusieurs conditions opératoires. Cette phase de conditionnement est réalisée dans le réacteur de réduction 2 ou dans une autre enceinte, dédiée à cette phase de conditionnement, différente du réacteur 2 et du réacteur de synthèse 4.

Après cette phase de conditionnement le catalyseur est envoyé dans le réacteur de synthèse 4 maintenu en opération. On parle ici d'appoint (en anglais make-up) en catalyseur réalisé sans interruption de la réaction de synthèse Fischer-Tropsch.

En effet, dans un procédé Fischer-Tropsch, on peut observer une baisse d'activité et de productivité soit par perte de catalyseur due à son attrition (et entrainement des fines de catalyseur) avec la sortie des produits formés, soit par désactivation du catalyseur au cours du temps. Ainsi, afin de maintenir la productivité de l'unité à son meilleur niveau, il est nécessaire de faire des appoints de catalyseur frais pour compenser les pertes de matière et/ou d'activité du catalyseur.

Grâce au procédé selon l'invention cet appoint est réalisée directement à partir du réacteur de réduction et/ou de conditionnement, soit en mode continu, soit en mode semi-continu (par batch) sans nécessiter un arrêt, même temporaire, de l'unité de synthèse. En outre les conditions de réduction/conditionnement sont optimisées afin de former un catalyseur réduit et conditionné dont l'activité catalytique est déjà adaptée pour fonctionner dans les conditions opératoires rencontrées dans le réacteur de synthèse FT, c'est à dire un catalyseur directement "prêt à l'emploi".

En résumé, il découle du procédé et de l'unité de fabrication selon l'invention les avantages suivants:
- moindre coût en terme de gestion du flux de matières car la réduction du précurseur du catalyseur est réalisée sur le site même de l'unité de synthèse, éventuellement avec une source de gaz réducteur qui est la même que celle utilisée pour la synthèse FT et qui est disponible en permanence;
- disposer d'un catalyseur actif et réduit dans des conditions optimales et qui présente des performances catalytiques améliorées et qui n'a pas subi des processus de stockage ou de protection;
- disposer à tout moment de catalyseur réduit et conditionné dans des conditions optimales et maîtrisées, directement prêt à l'emploi pour réaliser la synthèse Fischer-Tropsch et qui réduit (voire supprime) les phases transitoires de démarrage ou d'appoint.
- disposer d'une unité de synthèse FT dont les appoints de catalyseur "frais" ne nécessite pas une interruption de la réaction Fischer-Tropsch ou un arrêt de l'unité et sont réalisés en préservant le catalyseur

### Exemple 1

Un test a été effectué dans une installation pilote basée sur le schéma de principe de la Fig. 1. Le précurseur de catalyseur est un oxyde de cobalt supporté sur un support Siralox® (alumine stabilisée par Si et commercialisée par Sasol). La teneur en cobalt est de 13% en poids par rapport au poids du total du précurseur de catalyseur.

30g de précurseur du catalyseur sont chargés dans le réacteur de réduction et sont soumis à quatre traitements successifs, à savoir sous H₂ puis sous CO, et enfin sous gaz de synthèse dans un rapport molaire H₂/CO de 7 puis de 2. Les conditions sont précisées dans le tableau 1 suivant:

**Tableau 1**

| Traitement | N°1 (réduction) | N°2 (conditionnement 1) | N°3 (conditionnement 2) | N°4 (conditionnement 3) |
|---|---|---|---|---|
| Gaz réducteur | H₂ | CO (mélange CO/N₂) | H₂+CO | H₂+CO |
| T (°C) | 400°C | 200°C | 230°C | 230°C |
| P (MPa) | 0,1 | 3 | 2 | 2,6 |
| GHSV (Nl/h/g) | 2.5 | 2 | 5 | 5 |
| Pureté du gaz réducteur (% vol) | 100 | 10 (10/90) | 100 | 70% |
| Rapport H₂/CO | - | - | 7 | 2 |
| Durée | 4 h | 24 h | 8 h | 2 h |

A l'issue de ces traitements de réduction et conditionnement, le catalyseur ainsi activé est transféré dans le réacteur de synthèse Fischer-Tropsch (FT) de type slurry, préalablement chargé d'un solvant paraffinique (octadécane) et porté à 120°C.

Après chargement du catalyseur, le gaz de synthèse (dilution 30%, rapport molaire H₂/CO = 2.0) est introduit dans le réacteur de synthèse FT. La pression est portée à 26 bar et la température est porté à 230°C en suivant une rampe de 50°C/h jusqu'à 200°C puis de 10°C/h entre 200 et 230°C.

La réaction de synthèse FT est alors conduite en continue dans les conditions suivantes : 26 bar, 230°C, sous une alimentation en gaz de synthèse dilué à 30% dans N₂ (pureté du gaz de synthèse 70%) et caractérisé par un rapport molaire H₂/CO de 2.0 (soit composé à 1/3 de CO et 2/3 d'H₂). Le débit de gaz de synthèse est régulièrement ajusté de manière à maintenir le taux de conversion de CO à 56%.

En parallèle à l'opération du réacteur de synthèse Fischer-Tropsch, un nouveau lot de catalyseur est préparé dans le réacteur de réduction en suivant la même procédure que pour le premier batch préparé (cf tableau 1) : 3 g de précurseur de catalyseur sont chargés dans le réacteur de réduction et sont soumis aux quatre traitements successifs décrit dans le tableau 1.

Au bout de 30 jours de synthèse, ce second lot de catalyseur est transféré dans le réacteur de synthèse Fischer-Tropsch en cours d'opération, en mettant en oeuvre une différence de pression entre les deux réacteurs. Cet ajout de catalyseur en cours d'opération du réacteur de synthèse Fischer-Tropsch permet de ré-augmenter son niveau d'activité et sa productivité : l'appoint de catalyseur "frais" permet de ré-augmenter le débit de charge et accroître ainsi la production d'hydrocarbures Fischer-Tropsch.

Les performances catalytiques sont suivies au cours du temps (Tableau 2).

**Tableau 2**

| Temps (jour) | Chargement ou appoint de catalyseur (g) | T (°C) | P (MPa) | Débit de gaz de synthèse (Nl/h) | Conversion de CO (%) | Production de C5+ (g/h) |
|---|---|---|---|---|---|---|
| 0 | 30 | 230 | 2,6 | 150 | 47.8 | 6.9 |
| 1 | - | 230 | 2,6 | 120 | 56.1 | 6.5 |
| **8** | **-** | **230** | **2,6** | **104** | **56.2** | **5.6** |
| 15 | - | 230 | 2,6 | 98 | 56.0 | 5.3 |
| 29 | - | 230 | 2,6 | 95 | 56.2 | 5.1 |
| **30** | **3** | **230** | **2,6** | **104** | **56.3** | **5.6** |

Au démarrage, le catalyseur présente une sur-activité transitoire temporaire qui se traduit par une productivité importante (C5+ > 6 g/h).

Après 8 jours d'opération le système a atteint son régime stationnaire avec des valeurs de production C5+ quasiment stables. Entre le 8^{ième} et le 30^{ième} jour de fonctionnement, le débit de gaz de synthèse est ajusté afin de maintenir un taux de conversion de CO égal à environ 56%. Durant cette période, on peut considérer que le système est en régime stationnaire car la variation du débit (à taux de conversion CO constante) est plus faible et moins fréquente que par rapport aux huit premiers jours.

Au bout d'un mois on observe une légère diminution de la production de C5+ qui passe de 5.6 à 5.1 g/h. Un appoint de catalyseur est alors effectué le 30^{ème} jour. On note que la valeur du paramètre de production C5+ augmente rapidement pour retrouver une valeur correspondante à celle rencontrée après 8 jours de fonctionnement.

Cet exemple illustre d'une certaine manière l'opération d'une unité industrielle qui fonctionne à capacité constante.

### Exemple 2

### A) Réduction et conditionnement d'un catalyseur selon l'invention

Un test a été effectué dans une installation pilote basée sur le schéma de principe de la Fig. 1. Le précurseur de catalyseur est un oxyde de cobalt promu par du ruthénium et supporté sur un support Puralox® (de type alumine commercialisé par Sasol). Le catalyseur contient ainsi 11 % en poids de cobalt et 0.2 % poids de ruthénium par rapport au poids du précurseur de catalyseur.

30g de précurseur du catalyseur sont chargés dans le réacteur de réduction et sont soumis à un traitement sous gaz de synthèse, dont les conditions sont précisées dans le tableau 3 suivant :

**Tableau 3**

| Traitement | N°1 |
|---|---|
| Gaz réducteur | H₂+CO |
| T (°C) | 230°C |
| P (bar) | 30 |
| GHSV (Nl/h/g) | 0.6 |
| Pureté du gaz réducteur (% vol) | 100 |
| Rapport H₂/CO | 2.0 |
| Durée | 24 h |

A l'issue de ce traitement de réduction, le catalyseur ainsi activé est transféré dans le réacteur de synthèse Fischer-Tropsch (FT) de type slurry, préalablement chargé d'un solvant paraffinique (octadécane) et portée à 230°C. Préalablement au chargement du catalyseur, le gaz de synthèse de rapport H₂/CO 2.0 est introduit dans le réacteur de synthèse FT. La pression est portée à 20 bar. La réaction de synthèse FT est alors conduite en continue dans les conditions suivantes : 20 bar, 230°C, sous une alimentation en gaz de synthèse pur de 120 NL/h et dont le rapport H2/CO est de 2.0. Les performances catalytiques sont suivies au cours du temps (Tableau 2).

### B) Exemple comparatif

Le précurseur de catalyseur (30 g) utilisé dans l'exemple A) est réduit de façon conventionnelle, en présence d'hydrogène, dans une installation dédiée (ex situ), à 350°C pendant 8 h.

Après réduction, le catalyseur réduit est chargé dans le réacteur de synthèse Fischer-Tropsch et testé dans les mêmes conditions que dans l'exemple A) soit : 20 bar, 230°C, sous une alimentation en gaz de synthèse pur, ratio H2/CO de 2.0, débit 120 Nl/h. Les performances catalytiques sont mesurées au cours du temps (Tableau 4).

**Tableau 4 : Performances des catalyseurs A et B**

| Temps | Catalyseur | Conversion CO (%) | Sélectivité CH4 (% mol C) | Sélectivité C5+ (% mol C) |
|---|---|---|---|---|
| 2 h | A* | 50 | n.d. | n.d. |
| 2 h | B** | 10 | n.d. | n.d. |
| 24 h | A* | 51 | 6.5 | 85.5 |
| 24 h | B** | 45 | 8 | 82 |
| 1 semaine | A* | 50 | 7 | 84.5 |
| 1 semaine | B** | 44 | 8.5 | 81 |

| | | | | |
|---|---|---|---|---|
| * selon l'invention ** comparatif | | | | |

Les résultats obtenus, présentés dans le tableau 4, montrent l'intérêt de la procédure de réduction du catalyseur Fischer-Tropsch en présence d'un gaz réducteur comprenant un mélange H₂ et CO. Ainsi le catalyseur A réduit à basse température (230°C) et en présence de gaz de synthèse, disponible sur site, dans un équipement intégré au schéma du procédé Fischer-Tropsch, présente des performances catalytiques améliorées, à la fois en terme d'activité et de sélectivité. En outre, ce catalyseur est immédiatement opérationnel.

Notre invention permet de fournir un tel catalyseur activé en présence de gaz de synthèse pour le démarrage de l'unité Fischer-Tropsch et également pour tous les appoints en catalyseur en cours d'opération du réacteur de synthèse Fischer-Tropsch (non illustré dans cet exemple mais qui peut être réalisé comme dans l'exemple 1).

## Revendications

1. Procédé de fabrication en continu d'hydrocarbures à partir de gaz de synthèse en présence d'un catalyseur comprenant une étape de synthèse dans laquelle on fait réagir un gaz de synthèse en présence d'un catalyseur dans un réacteur de synthèse Fischer-Tropsch (4),
**caractérisé en ce que** simultanément à l'étape de synthèse et sur le site même de l'unité de synthèse Fischer-Tropsch, on effectue les étapes successives suivantes:
a) chargement d'un précurseur du catalyseur comprenant de l'oxyde de cobalt dans un réacteur de réduction (2) dédié;
b) réduction du précurseur du catalyseur chargé à l'étape a) par mise en contact avec un gaz réducteur comprenant de l'hydrogène (H₂) ou du monoxyde de carbone (CO), la réduction est effectuée à une température comprise entre 200 et 500°C, à une pression totale comprise entre 0,1 et 5 MPa et sous un flux gazeux compris entre 0,1 et 20 NI/h/g de catalyseur à traiter, pendant 1 à 24 heures;
c) au moins une étape de conditionnement du catalyseur réduit à l'étape b), le conditionnement étant effectué par mise en contact du catalyseur réduit avec un gaz réducteur comprenant du monoxyde de carbone seul ou un mélange d'hydrogène et de monoxyde de carbone dont le rapport molaire H₂/CO est compris entre 0,01 et 10, le conditionnement étant effectué à une température comprise entre 200 et 500°C, à une pression totale comprise entre 0,1 et 5 MPa et sous un flux gazeux compris entre 0,1 et 20 NI/h/g de catalyseur à traiter, pendant 1 à 48 heures; et
d) introduction du catalyseur réduit à l'étape c) dans le réacteur de synthèse (4) en mode semi-continu ou en mode continue.

2. Procédé selon la revendication 1, dans lequel le gaz réducteur comprenant un mélange d'hydrogène (H₂) et de monoxyde de carbone (CO), est issu d'une source de gaz de synthèse (1) qui alimente également le réacteur de synthèse (4).

3. Procédé de fabrication selon la revendication 2 comprenant une étape préalable de traitement du gaz de synthèse en amont de l'étape de réduction du précurseur de catalyseur de sorte à modifier le rapport molaire H₂/CO du gaz réducteur alimentant le réacteur de réduction (2).

4. Procédé selon l'une des revendications précédentes dans lequel le rapport molaire H₂/CO du gaz réducteur de l'étape b) est compris entre 0,01 et 10.

5. Procédé selon l'une des revendications précédentes dans lequel le précurseur de catalyseur inclue un support comprenant un oxyde d'un ou plusieurs éléments choisi parmi Al, Si, Ti, Zr, Ce, Cu, Zn, Ni, Co.

6. Procédé l'une des revendications précédentes, dans lequel le précurseur de catalyseur comprend en outre un ou plusieurs métaux ou oxyde(s) métallique(s) sélectionnés parmi le Cu, Mn, Ru, Pd, Pt, Re, La.

7. Procédé selon l'une des revendications précédentes, dans lequel le rapport molaire H₂/CO du gaz réducteur de conditionnement est compris entre 1 et 2,5.

8. Procédé selon l'une des revendications précédentes, dans lequel l'étape de conditionnement comprend une pluralité de phases de conditionnement, chaque phase étant réalisée en présence d'un gaz réducteur de conditionnement ayant un rapport molaire H₂/CO différent de celui de la phase qui la précède.

9. Procédé selon l'une des revendications précédentes, dans lequel l'étape de conditionnement du catalyseur est réalisée dans le réacteur de réduction du précurseur de catalyseur.

10. Procédé selon l'une des revendications 1 à 8, dans lequel l'étape de conditionnement du catalyseur est réalisée dans un réacteur dédié, différent du réacteur de réduction du précurseur de catalyseur et différent du réacteur de synthèse Fischer-Tropsch.

11. Procédé selon l'une quelconque des revendications précédentes dans lequel l'alimentation du catalyseur réduit dans le réacteur de synthèse Fischer-Tropsch est réalisé au moyen d'un changement de pression.

## Patentansprüche

1. Verfahren zur kontinuierlichen Herstellung von Kohlenwasserstoffen aus Synthesegas in Anwesenheit eines Katalysators, umfassend einen Schritt der Synthese, in dem ein Synthesegas in Anwesenheit eines Katalysators in einem Fischer-Tropsch-Synthesereaktor (4) umgesetzt wird,
**dadurch gekennzeichnet, dass** gleichzeitig mit dem Syntheseschritt und am Ort der Fischer-Tropsch-Syntheseeinheit selbst, die folgenden Schritte durchgeführt werden:
a) Laden eines Vorläufers des Katalysators, der Kobaltoxid umfasst, in einen dedizierten Reduktionsreaktor (2);
b) Reduktion des Vorläufers des Katalysators, der in Schritt a) geladen wird, durch Inkontaktbringen mit einem Reduktionsgas, das Wasserstoff (H₂) oder Kohlenmonoxid (CO) umfasst, wobei die Reduktion bei einer Temperatur zwischen 200 und 500°C, bei einem Gesamtdruck zwischen 0,1 und 5 MPa und unter einem Gasstrom zwischen 0,1 und 20 Nl/h/g des zu behandelnden Katalysators während 1 bis 24 Stunden bewirkt wird;
c) mindestens ein Konditionierungsschritt des in Schritt b) reduzierten Katalysators, wobei die Konditionierung durch Inkontaktbringen des reduzierten Katalysators mit einem Reduktionsgas bewirkt wird, das nur Kohlenmonoxid oder ein Gemisch von Wasserstoff und Kohlenmonoxid umfasst, dessen Molverhältnis H₂/CO zwischen 0,01 und 10 liegt, wobei die Konditionierung bei einer Temperatur zwischen 200 und 500°C, bei einem Gesamtdruck zwischen 0,1 und 5 MPa und unter einem Gasstrom zwischen 0,1 und 20 Nl/h/g des zu behandelnden Katalysators während 1 bis 48 Stunden bewirkt wird; und
d) Einbringen des in Schritt c) reduzierten Katalysators in den Synthesereaktor (4) im semikontinuierlichen Modus oder im kontinuierlichen Modus.

2. Verfahren nach Anspruch 1, wobei das Reduktionsgas, das ein Gemisch von Wasserstoff (H₂) und Kohlenmonoxid (CO) umfasst, von einer Synthesegasquelle (1) stammt, die auch den Synthesereaktor (4) speist.

3. Herstellungsverfahren nach Anspruch 2, umfassend einen vorherigen Schritt der Behandlung des Synthesegases stromaufwärts von dem Schritt der Reduktion des Katalysatorvorläufers, um das Molverhältnis H₂/CO des Reduktionsgases zu modifizieren, das den Reduktionsreaktor (2) speist.

4. Verfahren nach einem der vorhergehenden Ansprüche, wobei das Molverhältnis H₂/CO des Reduktionsgases aus Schritt b) zwischen 0,01 und 10 liegt.

5. Verfahren nach einem der vorhergehenden Ansprüche, wobei der Katalysatorvorläufer einen Träger umfasst, der ein Oxid eines oder mehrerer Elemente umfasst, ausgewählt aus Al, Si, Ti, Zr, Ce, Cu, Zn, Ni, Co.

6. Verfahren nach einem der vorhergehenden Ansprüche, wobei der Katalysatorvorläufer außerdem ein oder mehrere Metalle oder Metalloxide umfasst, ausgewählt aus Cu, Mn, Ru, Pd, Pt, Re, La.

7. Verfahren nach einem der vorhergehenden Ansprüche, wobei das Molverhältnis H₂/CO des Reduktionsgases der Konditionierung zwischen 1 und 2,5 liegt.

8. Verfahren nach einem der vorhergehenden Ansprüche, wobei der Schritt der Konditionierung mehrere Konditionierungsphasen umfasst, wobei jede Phase in Anwesenheit eines Reduktionsgases der Konditionierung mit einem Molverhältnis H₂/CO durchgeführt wird, das von jedem der Phase verschieden ist, die ihr vorausgeht.

9. Verfahren nach einem der vorhergehenden Ansprüche, wobei der Schritt der Konditionierung des Katalysators im Reduktionsreaktor des Katalysatorvorläufers durchgeführt wird.

10. Verfahren nach einem der Ansprüche 1 bis 8, wobei der Schritt der Konditionierung des Katalysators in einem dedizierten Reaktor durchgeführt wird, der von dem Reduktionsreaktor des Katalysatorvorläufers verschieden ist und von dem Fischer-Tropsch-Synthesereaktor verschieden ist.

11. Verfahren nach einem der vorhergehenden Ansprüche, wobei die Speisung des Katalysators, der in dem Fischer-Tropsch-Synthesereaktor reduziert wird, mittels einer Druckänderung durchgeführt wird.

## Claims

1. Method for the continuous production of hydrocarbons from synthesis gas in the presence of a catalyst comprising a synthesis step in which a synthesis gas is reacted in the presence of a catalyst in a Fischer-Tropsch synthesis reactor (4),
**characterised in that**, at the same time as the synthesis step and at the actual site of the Fischer-Tropsch synthesis unit, the following successive steps are carried out:
a) charging a catalyst precursor comprising cobalt oxide in a dedicated reduction reactor (2);
b) reducing the catalyst precursor charged in step a) by placing it in contact with a reduction gas comprising hydrogen (H₂) or carbon monoxide (CO), the reduction being carried out at a temperature between 200 and 500°C, at a total pressure between 0.1 and 5 MPa and under a gas flow between 0.1 and 20 NI/h/g of catalyst to be processed, during 1 to 24 hours;
c) at least one step for conditioning the catalyst reduced in step b), the conditioning operation being carried out by placing the reduced catalyst in contact with a reduction gas which comprises carbon monoxide alone or an admixture of hydrogen and carbon monoxide wherein the molar ratio H₂/CO is between 0.01 and 10, the conditioning operation being carried out at a temperature between 200 and 500°C, at a total pressure between 0.1 and 5 MPa and under a gas flow between 0.1 and 20 Nl/h/g of catalyst to be processed, during 1 to 48 hours; and
d) introducing the catalyst reduced in step c) into the synthesis reactor (4) in semi-continuous mode or in continuous mode.

2. Method according to claim 1, wherein the reduction gas which comprises an admixture of hydrogen (H₂) and carbon monoxide (CO), originates from a source of synthesis gas (1) which also supplies the synthesis reactor (4).

3. Production method according to claim 2 comprising a prior step for processing the synthesis gas upstream of the step for reducing the catalyst precursor in order to modify the molar ratio H₂/CO of the reduction gas which supplies the reduction reactor (2).

4. Method according to any one of the preceding claims wherein the molar ratio H₂/CO of the reduction gas of step b) is between 0.01 and 10.

5. Method according to any one of the preceding claims wherein the catalyst precursor includes a support which comprises an oxide of one or more elements selected from Al, Si, Ti, Zr, Ce, Cu, Zn, Ni, Co.

6. Method according to any one of the preceding claims, wherein the catalyst precursor further comprises one or more metals or metal oxide(s) selected from Cu, Mn, Ru, Pd, Pt, Re, La.

7. Method according to any one of the preceding claims, wherein the molar ratio H₂/CO of the conditioning reduction gas is between 1 and 2.5.

8. Method according to any one of the preceding claims, wherein the conditioning step comprises a plurality of conditioning phases, each phase being carried out in the presence of a conditioning reduction gas which has a molar ratio H₂/CO which is different from that of the preceding phase.

9. Method according to any one of the preceding claims, wherein the step for conditioning the catalyst is carried out in the reactor for reduction of the catalyst precursor.

10. Method according to any one of claims 1 to 8, wherein the step for conditioning the catalyst is carried out in a dedicated reactor which is different from the reactor for reduction of the catalyst precursor and different from the Fischer-Tropsch synthesis reactor.

11. Method according to any one of the preceding claims wherein the supply of the reduced catalyst to the Fischer-Tropsch synthesis reactor is carried out by means of a pressure change.
